(19) Europäisches Patentamt
European Patent Office
Office européen des brevets

(11) **EP 2 271 439 B1**

(12) **EUROPÄISCHE PATENTSCHRIFT**

(45) Veröffentlichungstag und Bekanntmachung des Hinweises auf die Patenterteilung:
**23.12.2015  Patentblatt 2015/52**

(21) Anmeldenummer: **08867807.3**

(22) Anmeldetag: **23.10.2008**

(51) Int Cl.:
***B06B 1/06*** *(2006.01)*

(86) Internationale Anmeldenummer:
**PCT/EP2008/008954**

(87) Internationale Veröffentlichungsnummer:
**WO 2009/083053 (09.07.2009 Gazette 2009/28)**

(54) **MULTIFREQUENZ-SENDE- UND EMPFANGSEINHEIT FÜR MEDIENGEBUNDENE WELLEN**

MULTI-FREQUENCY TRANSMITTING AND RECEIVING UNIT FOR WAVES BOUND TO MEDIA

UNITÉ D'ÉMISSION ET DE RÉCEPTION MULTIFRÉQUENCE DESTINÉE À DES ONDES LIÉES À DES SUBSTANCES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.12.2007  DE 202007017913 U**

(43) Veröffentlichungstag der Anmeldung:
**12.01.2011  Patentblatt 2011/02**

(73) Patentinhaber: **Yucoya Energy Safety GmbH**
**97970 Würzburg (DE)**

(72) Erfinder: **FRITSCH, Dr. Thomas**
**97286 Sommerhausen (DE)**

(74) Vertreter: **Kohler Schmid Möbus Patentanwälte**
**Partnerschaftsgesellschaft mbB**
**Kaiserstrasse 85**
**72764 Reutlingen (DE)**

(56) Entgegenhaltungen:
**EP-A1- 0 062 477     EP-A1- 0 289 382**
**EP-A2- 0 397 961     DE-A1- 3 311 040**
**US-A- 4 437 348      US-A- 4 569 231**

## Beschreibung

Die vorliegende Erfindung betrifft eine Ultraschall-Sende- und -Empfangseinheit mit mindestens einem Wandler zur Erzeugung von Mehrfrequenz-Ultraschallsignalen und zum Empfang von durch ein Untersuchungsmedium beeinflussten, insbesondere reflektierten, gedämpften oder abgelenkten Ultraschallsignalen.

Bekannte Ultraschall-Sendeeinheiten bestehen üblicherweise aus einem oder mehreren, z. B. in einer Matrix angeordneten Piezokristallen, welche durch eine entsprechende elektrische Erregung ein Ultraschallsignal mit einer bestimmten Frequenz abgeben. Typische Frequenzen liegen in einem Frequenzband von z. B. 1 MHz - 8 MHz. Hierbei sind für den Kristall bezogen auf die abgegebene Frequenz charakteristische räumliche und elektrische Parameter maßgeblich.

Sowohl in der medizinischen Diagnostik als auch in der zerstörungsfreien Materialprüfung, dem "nondestructive testing" unterschiedlicher Materialien oder Materialkompositionen, gibt es zahlreiche Anwendungsmöglichkeiten für derartige Vorrichtungen. So besteht beispielsweise ein Hauptproblem in der Medizin darin, ein bestimmtes räumliches Gebiet im menschlichen Körper als Ganzes mittels Ultraschall zu erfassen. Üblicherweise werden hierzu nur Ultraschallsonden mit einer einzigen Frequenz verwendet. Das grundsätzliche Problem besteht hierbei darin, dass Wellenlänge und Frequenz zueinander in einer reziproken Beziehung gemäß der Gleichung

$$c\_m = \lambda * f$$

stehen, wobei c_m die Ausbreitungsgeschwindigkeit des Schalls im jeweiligen Medium bezeichnet, λ die Wellenlänge in diesem Medium und f die Frequenz, mit der das Ultraschallsignal ausgesandt wird. Da die Wellenlänge umgekehrt proportional zur Frequenz ist, wird bei gegebener Frequenz die effektive Eindringtiefe durch die Abschwächungseigenschaften des untersuchten beschallten Mediums bestimmt. Ist es für den jeweiligen diagnostischen Vorgang daher erforderlich, aus Gründen der Untersuchungsgüte eine andere Frequenz zu wählen, um eine andere Eindringtiefe zu erzielen, dann wird herkömmlicherweise die verwendete Ultraschall-Sendeeinheit durch eine für die neu gewählte Frequenz besser geeignete ausgetauscht. So werden z. B. für Untersuchungen der Oberflächenstruktur der Haut sehr hohe Frequenzen bis zu 30 MHz eingesetzt.

Der Einsatz von Ultraschallsonden mit der Eigenschaft, Ultraschallsignale mit verschiedenen Frequenzen aussenden zu können, wie beispielsweise aus der DE 297 08 338 U1 bekannt, erlaubt es, bei niedrigeren Frequenzen eine größere Eindringtiefe und bei höheren Frequenzen eine größere räumliche Auflösung zu erzielen. Üblicherweise will man aber in den meisten Fällen *beides gleichzeitig* erreichen, ist aber durch die auf dem Markt erhältlichen Geräte dazu gezwungen, fast immer einen Kompromiss eingehen zu müssen, der dem diagnostischen Ziel oft nicht gerecht wird.

In anderen Einsatzgebieten der Ultraschalldiagnostik wie der zerstörungsfreien Materialprüfung bzw. "nondestructive testing" (NDT) besteht das Ziel darin, mittels Ultraschall interessierende Eigenschaften des betreffenden Materials (des beschallten Mediums) in Erfahrung zu bringen. Hierfür sind Ultraschallsonden mit einer für das jeweilige Medium geeigneten Frequenz verwendbar. Die Frequenzbereiche reichen hierbei von niedrigen Frequenzen mit mehreren 100 kHz bis zu Frequenzen im zweistelligen MHz-Bereich, je nachdem, welche Materialeigenschaften man feststellen will. Zu untersuchende Medien können feste Materialien wie Metalle oder Fluide in flüssiger oder gasförmiger Phase sein aber auch gemischte Medien wie Emulsionen oder in Flüssigkeiten (z. B. in Öl) gelöste Gase. Insbesondere für kontinuierlich ablaufende chemische oder biologische Prozesse in Fluiden stellt die Detektion der unterschiedlichen Eigenschaften dieser Fluide wie auch von deren Zusammensetzung aus unterschiedlichen Komponenten, z. B. in Mischungen und Emulsionen oder von in diesen Fluiden gelösten festen oder gasförmigen Substanzen mittels Ultraschall eine große technische Herausforderung dar.

Es sind auch bereits ringförmig konzipierte Ultraschallerzeuger ("annular arrays") bekannt (siehe Sverre Holm, 1995), die im Allgemeinen mit nur einer Frequenz arbeiten. Diese Ultraschallsonden werden vorteilhafterweise in der medizinischen Diagnostik eingesetzt, um an für herkömmliche Ultraschallsonden schwer zugänglichen Orten im menschlichen Körper, z. B. im Vaginal- oder Rektalbereich, gute Ultraschallbilder der dort vorhandenen Gewebe oder Organe zu ermöglichen.

Die Erfordernisse der praktischen Anwendung insbesondere in der Medizin bedingen zumeist Ausführungsformen der Sonden, bei denen eine Fokussierung vorgesehen ist. Diese kann mechanisch oder dynamisch mittels Electronic Focusing erfolgen. Dies ergibt sich zum Einen aus der über das effektive Schallfeld (Fernfeld) einer Ultraschallsonde für eine bestimmte Frequenz festgelegte Eindringtiefe bei variierender Abschwächung (attenuation) des Signals durch das durchdrungene Gewebe und zum Anderen aus der durch die Wellenlänge vorgegebene Auflösung der vorgefundenen Gewebestrukturen. Da die Beziehung c = λ * f, d. h. Ausbreitungsgeschwindigkeit im Medium = Wellenlänge * Frequenz eine Reziprozität des Verhältnisses von Wellenlänge zu Frequenz bedingt, versucht man durch bestimmte technische Maßnahmen bei konstanter Frequenz mittels einer Fokussierung wenigstens in einem bestimmten Bereich, der so genannten Fokalzone, die Signalqualität zu verbessern.

Wird ein Ultraschallerzeuger, üblicherweise ein Piezokristall-Plättchen (bzw. eine rechteckige oder auch ringförmige Anordnung mehrerer kleinerer Plättchen, d. h. ein Array von Sub-Elementen) verwendet, dann ist der Durchmesser D des Erzeugers oder des Arraysmit der Wellenlänge über die Beziehung

$$\text{„Höhe (Länge, Ausdehnung) des Nahfeldes“} = D^2 / 2*\lambda$$

verknüpft. Das Nahfeld ist hierbei der Bereich in der Nähe des Erzeugers, in dem sich Interferenzen der ausgesandten Schallwellen derart herausbilden, dass diese zu einer "Unordnung" des Schallfelds in der Nähe des Transducers führen (Nahfeld, Fraunhofer-Zone). Hieraus empfangene Ultraschall-Signal-Bestandteile würden keiner sinnvoll zu interpretierenden Material- oder Medieninformation mehr entsprechen. Man muss sich daher beim Empfang von Ultraschallsignalen auf die Ultraschallwellen jenseits des Transitionspunktes bei

$$D^2/2*\lambda = D^2 * f / ( 2* c\_m) \qquad\qquad (1)$$

dem so genannten Fernfeld zurückgreifen, um aussagekräftige Informationen über das beschallte Medium zu erhalten. Entscheidend ist hierbei, dass auf Empfangsseite eine homogene und kohärente Wellenfront eintrifft. D und f stehen gemäß Gleichung (1) in einer reziproken Beziehung zueinander, d. h. mit wachsender Frequenz f wird in diesem Fall D immer kleiner, wenn das Nahfeld möglichst gleich groß (klein) bleiben soll, damit die dort auftretenden Interferenzen den Empfang der ausgesandten Ultraschallwellen bei der Empfängersonde nicht stören.

Aus der US 4,569,231 ist eine Ultraschalleinrichtung mit ringförmigen Piezoelementen bekannt, die Schallwellen unterschiedlicher Frequenzen erzeugen.

Mit dieser Einrichtung wird zielgerichtet der Umstand ausgenutzt, dass für unterschiedliche Frequenzen für unterschiedliche Ringe des Arrays verschieden tief im Schallfeld gelegene Fokuspunkte erzeugt werden, die dem oben genannten Zweck der Ermöglichung von hinreichend guten Ultraschallbildern im menschlichen Körper dadurch dienen, dass bei diesen frequenzbedingt verschiedenen Fokuspunkten entsprechend unterschiedliche Eindringtiefen bei vertretbarer Signalqualität ermöglicht werden. Dabei werden jeweils nur die reflektierten Signale des Sendesignals derjenigen Frequenz ausgewertet, die bei der gewünschten Eindringtiefe im Gewebe einen Fokuspunkt aufweisen. Das sich durch die Anordnung gemäß der US 4,569,231 ergebende Schallfeld ist bezüglich der Verteilung der Feldbereiche, in denen Signale mit allen verwendeten Frequenzen auftreten, äußerst inhomogen strukturiert, sodass die Auswertung der reflektierten Signale der anderen Frequenzen die Diagnose verfälschen würden. Zudem hängt die technische Konstruktion dieses bekannten ringförmigen Arrays fest von der Vorgabe der interessierenden Eindringtiefen, d. h. den Fokuszonen für die unterschiedlichen Frequenzen ab.

Aus der US 4,437,348 ist ebenfalls eine Ultraschalleinrichtung mit ringförmigen Piezoelementen bekannt, die Schallwellen unterschiedlicher Frequenzen erzeugen. Auch hier werden die Schallwellen fokussiert.

Der vorliegenden Erfindung liegt die Aufgabe zugrunde, eine Multifrequenz-Ultraschall-Sende- und -Empfangseinheit zu schaffen, mit der eine umfassende, alle Eigenschaften der relevanten physikalisch-chemischen Änderungsprozesse des untersuchten Mediums erfassende Detektion möglich ist.

Die Aufgabe wird mit einer Ultraschall-Sende- und -Empfangseinheit mit den Merkmalen des Anspruchs 1 gelöst. Die Unteransprüche geben vorteilhafte Weiterbildungen an.

Die Art der Anordnung der Elemente des mindestens einen Wandlers um ein gemeinsames Zentrum herum, das gleichzeitige Senden aller Elemente und die gleichzeitige Erfassung und Auswertung der durch das Untersuchungsmedium beeinflussten Signale aller Frequenzen ermöglicht eine weitgehende Erfassung aller interessierenden Eigenschaften des Untersuchungsmediums. Dabei ist es von Vorteil, wenn die Elemente die Ultraschallsignale kontinuierlich emittieren, um auch die Detektion von Änderungsprozessen des untersuchten Mediums zu ermöglichen.

Da der Öffnungswinkel (Apertur) des Schallfeldes eines Ultraschallerzeugers mit dem Durchmesser D von der jeweils verwendeten Frequenz gemäß der Beziehung

$$\text{Sin } \Theta := 1{,}22 * \lambda / D$$

mit $\theta$ als Öffnungswinkel und $\lambda < D$ als Wellenlänge abhängt, treten für hohe Frequenzen kleine Öffnungswinkel und für kleinere Frequenzen größere Öffnungswinkel auf. Dies hat zur Folge, dass die resultierenden, für den Empfang relevanten Fern-Schallfelder umso schmaler sind je höher die Frequenz ist. Dies bedeutet einerseits, dass lokal gebundene Prozesse, die im (weiteren) Schallfeld bei Aussendung einer niedrigeren Frequenz erfasst werden können, ggf. im Schallfeld einer höheren Frequenz nicht auftauchen. Es ist daher von Vorteil, wenn die äußeren Elemente Ultraschallsignale einer niedrigeren Frequenz emittieren als das zentrale Element, um zuverlässige Analysen durchführen zu können. Dabei kann das zentrale Element kreisförmig ausgebildet und das mindestens eine weitere Element ringförmig ausgebildet und konzentrisch zum zentralen Element angeordnet sein. Die Ringform und die konzentrische Anordnung führen zu annähernd kegelförmigen Abstrahlcharakteristiken der einzelnen Elemente.

Eine Anordnung der Elemente in einer Matrix wie im deutschen Gebrauchsmuster DE 297 08 338 U1 kann keine gleichmäßige Abstrahlcharakteristik erreichen. Oder es müssten, wie im dortigen Beispiel angegeben, die verwendeten Frequenzen so nahe beieinander liegen, dass nicht mehr von einer echten Multi-Frequenz-Ultraschallwelle gesprochen werden kann.

Die besten Ergebnisse lassen sich erzielen, wenn der Durchmesser der Elemente und die Breiten der ringförmigen Elemente sowie die Frequenzen der von den einzelnen Elementen emittierten Ultraschallsignale derart gewählt sind, dass in einem vorgegebenen Abstandsbereich zur Ultraschall-Sende- und -Empfangseinheit eine homogene Wellenfront der Ultraschallsignale aller Elemente entsteht.

Prinzipiell könnte auch eine gleichzeitige Verwendung mehrerer räumlich getrennter Ultraschalleinheiten mit jeweils unterschiedlichen Frequenzen erfolgen. Dies ist dann vorteilhaft, wenn die zu untersuchenden physikalisch-chemischen oder biologischen Prozesse mehrheitlich lokalisiert stattfinden. Tatsächlich sind aber die meisten Prozesse, die für eine derartige Untersuchung von Interesse sind, nicht notwendigerweise lokal zu begrenzen und weisen auch nicht an jedem Punkt des betrachteten Volumens die gleichen charakteristischen Merkmale auf. Als Beispiel hierfür mag die Biergärung dienen, die vornehmlich am Rande des Gärungsbehälters beginnt und sich nach innen fortsetzt. Es würde also eine Verfälschung und Missinterpretation der Daten stattfinden, wenn nicht alle mittels multifrequentem Ultraschall detektierbaren durch das Medium hervorgerufenen Signalveränderungen berücksichtigt würden, die zur Charakterisierung des jeweiligen Prozesses und der betreffenden Materialeigenschaften benötigt werden. Diese Signalveränderungen des Ultraschallsignals sind u. a. von der Verteilung der verschiedenen Substanzen im Messvolumen abhängig sowie von der Topologie bzw. Geometrie des zu untersuchenden Gebietes im Messvolumen.

Dies bedeutet, dass bei der räumlichen Trennung der Wandler Fehler auftreten, bedingt durch die Art der Signalausbreitung im Messvolumen, die mit

- der Geometrie/Topologie des Messraumes,

- der Anordnung der Wandler,

- deren Frequenzen und damit deren räumlichen Abstrahlcharakteristiken (i. e. Schallfelder)

verknüpft sind.

Die Ausbreitung der Ultraschallwellen kann hier zwar ebenfalls in kontinuierlicher Weise erfolgen, aber jeder Wandler sendet dabei nur *eine* Welle aus, die sich folglich mit denjenigen Wellen der anderen Wandler zumindest teilweise überlagern oder auslöschen und schließlich, abhängig von der Geometrie des Messraumes, Reflexionen und Beugungen erfahren können. Daher ist es plausibel, dass auch nicht bei jeder Geometrie des Messraumes alle ggf. interessierenden Gebiete gleichermaßen gut erfasst werden können. Eine "starre" Anordnung produziert naturgemäß Fehler bei ggf. erforderlichen flexiblen Messsituationen, sowohl in räumlicher als auch in zeitlicher Hinsicht. Dies bedeutet, dass problemabhängig die Position der Wandler variiert werden müsste. Es ist einleuchtend, dass dies wenig praktikabel ist und eine erhebliche Vorarbeit erforderlich sein würde, um eine für ein interessierendes Gebiet geeignete Schallausleuchtung jeweils immer neu zu finden. Die Vorteile der Multifrequenz werden somit zunehmend nur in eingeschränkter Weise wirksam, je komplexer die Messaufgabe ist. Dies gilt auch für den Fall, dass beispielsweise adaptiv eine für die jeweilige Messsituation "geeignete" Frequenz mit der ihr eigenen Abstrahlcharakteristik (bei sonst gleichem Aufbau der Wandler) gewählt wird.

Wird dagegen ein bestimmtes interessierendes Gebiet dreidimensional kontinuierlich mit multispektralem Ultraschall (i. e. mehreren geeigneten) Frequenzen derart beaufschlagt, dass einerseits die Abstrahlcharakteristik zu jedem Zeitpunkt konstant bleibt bei Verwendung einer Vielzahl von Frequenzen und dass andererseits den Empfangswandler eine kohärente Wellenfront erreicht, können unterschiedliche Eigenschaften und Prozesse des zu untersuchenden Mediums gleichmäßig gut dekretiert werden. Zudem können die Parameter der Ultraschall-Sende- und -Empfangseinheit derart gewählt werden, dass ein möglichst großer Bereich des resultierenden Schallfelds, insbesondere des Fernfelds, in homogener Weise von allen verwendeten Ultraschallsignalen mit unterschiedlichen Frequenzen überdeckt werden.

Bei der erfindungsgemäßen Ultraschall-Sende- und -Empfangseinheit findet keinerlei Fokussierung statt, sei es durch eine geometrische Verformung (Krümmung) oder in elektronischer Form. Dies wäre dem nachfolgend beschriebenen Ziel vollkommen abträglich.

Das Bestreben besteht darin, ein möglichst homogenes Fernfeld zu erzeugen, sodass an der Empfangseinheit eine kohärente Wellenfront auftrifft. Gleichzeitig soll möglichst jeder Punkt im dreidimensionalen Ausbreitungsraum der Ultraschallwellen (Fernfeld) von diesen mit allen verwendeten Frequenzen erreicht wird. Weiter soll die Gesamtcharakteristik des ausgestrahlten Schallfelds für alle verwendeten Frequenzen konstant gehalten werden, was wesentlich zur erwünschten Homogenität des Schallfeldes beiträgt.

Wichtig für die erfindungsgemäße Ultraschall-Sende- und -Empfangseinheit ist die Gewährleistung einer im Fernfeld gleich bleibenden Gesamtabstrahlcharakteristik bei der gleichzeitigen Verwendung mehrerer Frequenzen. Hierbei werden im Unterschied zur Vorrichtung der US 4,569,231 die Ultraschallwellen unfokussiert und kontinuierlich ausgesandt, wobei die für die zugeordneten Ringe ausgewählten Frequenzen gleichzeitig verwendet werden. Im Unterschied zum DE 297 08 338 U1 werden die Wandlerelemente nicht im "Resonanz-Betrieb" verwendet, sondern jedem Einzel-Ring bzw. einer Gruppe von Einzel-Ringen kann eine separate Frequenz zugeordnet werden. Es kommt nun auf die Kombination von bestimmten Frequenzen für die einzelnen Ringe an, um im Fernfeld-Bereich eine homogene Überdeckung einer bestimmten "region of interest" (ROI) mit multifrequentem Ultraschall zu jedem beliebigen Zeitpunkt in kohärenter Weise zu erzielen.

Die Flexibilität der erfindungsgemäßen Ultraschall-Sende- und -Empfangseinheit lässt sich dadurch erhöhen, dass mehrere ringförmige Elemente vorgesehen sind, deren Breite und/oder deren gegenseitiger Abstand variiert. Dies ermöglicht die Verwendung anderer Frequenzen als bei einem Array mit gleichmäßig breiten und gleichmäßig beabstandeten Ringen, ohne das Erreichen des vorstehend beschriebenen Ziels zu beeinträchtigen.

Die ringförmigen Elemente können auch mindestens teilweise in dicht benachbarte Einzelringe aufgeteilt sein, die jeweils Ultraschallsignale geringer Frequenzdifferenz emittieren. Mit einem solchen ringförmigen Element kann somit ein ganzes Frequenzband abgedeckt werden.

Die Elemente des Wandlers sind vorzugsweise Piezoelemente. Dabei kann die Dicke der ringförmigen Elemente größer sein als die Dicke des zentralen Elements.

Weiter ist es von Vorteil, wenn eine optische oder akusto-optische Empfangseinrichtung vorgesehen ist. Eine solche Empfangseinrichtung ist beispielsweise in der DE 197 32 968 A1 beschrieben. Sie hat den Vorteil einer gleichmäßigen, flachen Empfangscharakteristik über eine große Frequenz-Bandbreite, die den Gebrauch mehrerer Frequenzen auf der Senderseite optimal unterstützt.

Nachfolgend wird die Erfindung anhand der Zeichnung näher erläutert.

Es zeigen:

Fig. 1         Abstrahlcharakteristiken eines Ultraschallwandlers des Durchmessers D = 10 mm bei drei unterschiedlichen Frequenzen;

Fig. 2a - 2c   Abstrahlcharakteristiken einer Ultraschall-Sende- und -Empfangseinheit mit einem zentralen Element und zwei ringförmigen Elementen bei denselben drei Frequenzen wie in Figur 1.

Fig. 3 eine schematische Darstellung eines Ultraschallwandlers mit ringförmiger Anordnung der Wandlerelemente;

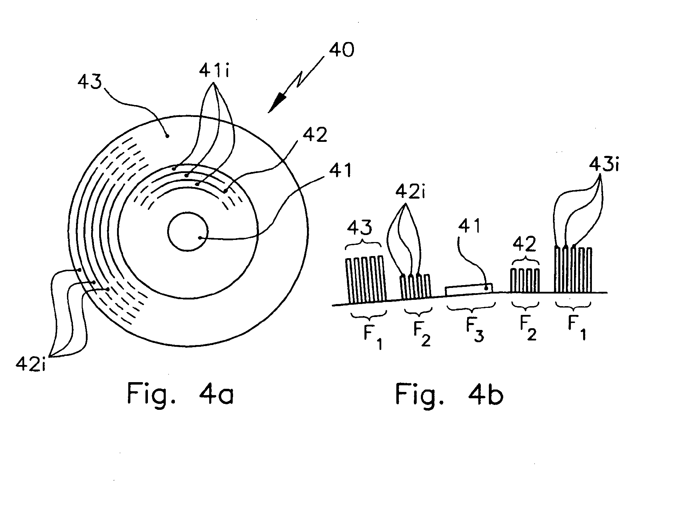

Fig. 4a, b eine schematische Darstellung eines Ultraschallwandlers mit ringförmigen Wandlerelementen, sowie einen Querschnitt durch diesen Wandler.

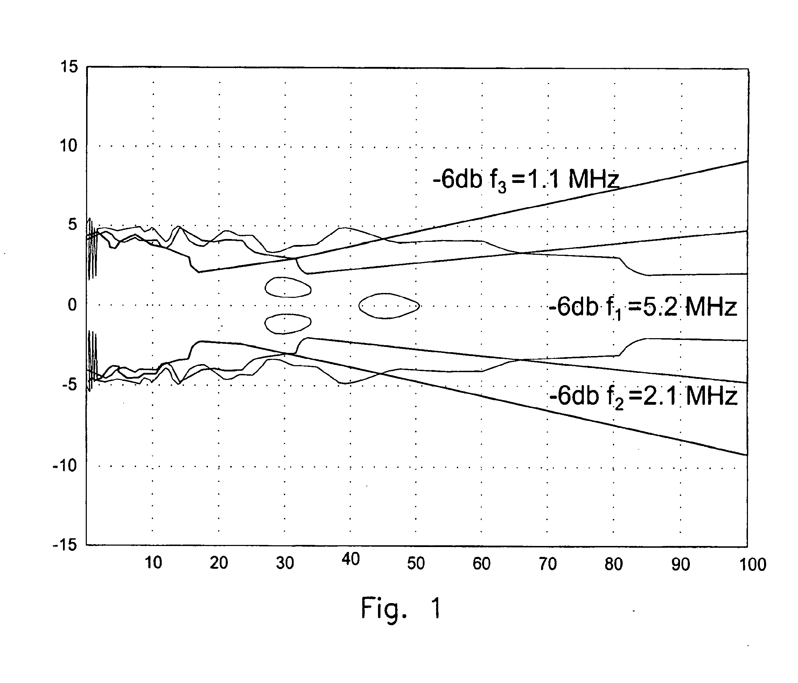

Figur 1 illustriert an einem Wandler mit einem kreisförmigen Element eines Durchmessers von D = 10 mm, dass für hohe Frequenzen kleine Öffnungswinkel der Abstrahlcharakteristiken auftreten und für kleinere Frequenzen größere Öffnungswinkel. Dies bedeutet, dass die resultierenden für den Empfang relevanten Fern-Schallfelder umso schmäler sind je höher die Frequenz ist.

In Figur 1 sind für den kreisförmigen Wandler mit Durchmesser 10mm für drei verschiedene Frequenzen $f\_1$ = 5,2 MHz, $f\_2$ = 2,1 MHz und $f\_3$ = 1,1 MHz die jeweilige Schallfeld-Konturen für -6 db angegeben. Man erkennt deutlich, dass sich das Schallfeld für höhere Frequenzen immer stärker "einengt". Die überlagerte Gesamtabstrahlcharakteristik für die drei Frequenzen ist nicht homogen. Für die Ermittlung der Schallfelder wurde zugrunde gelegt, dass keine Fokussierung erfolgt.

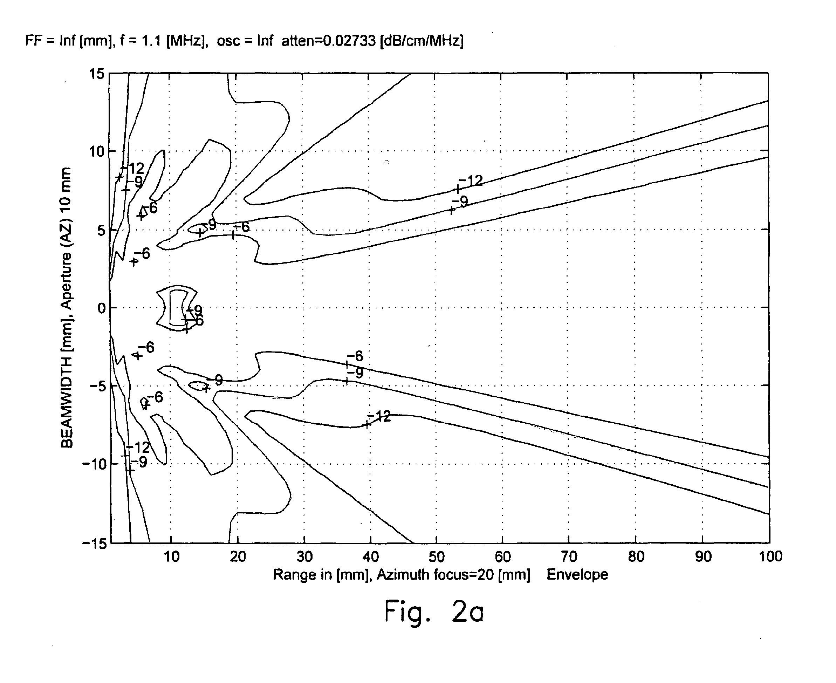

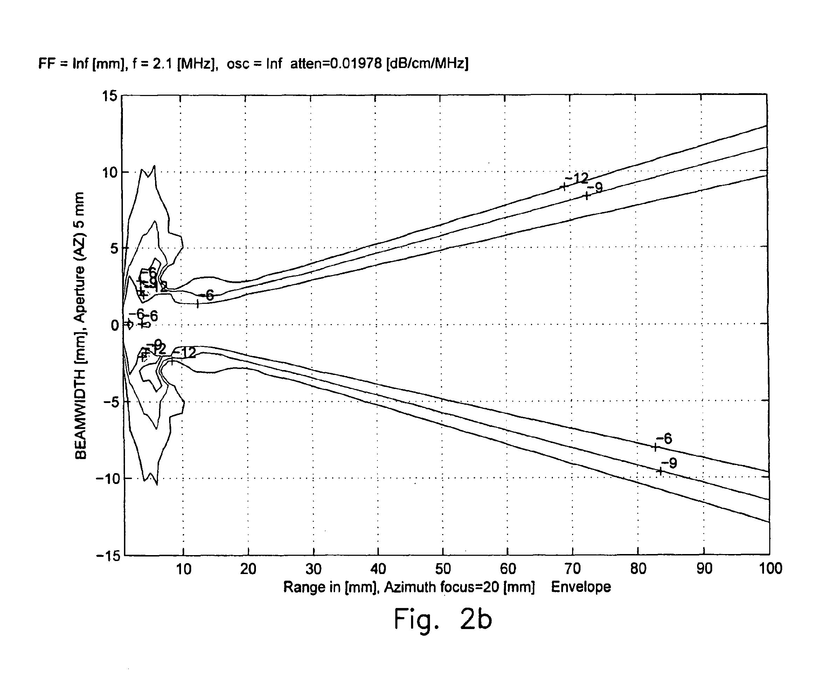

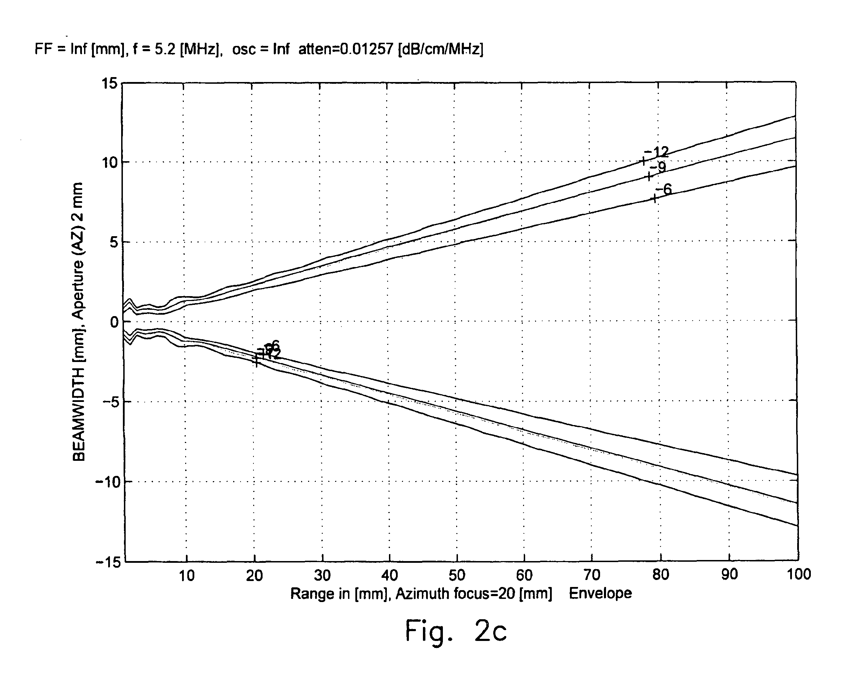

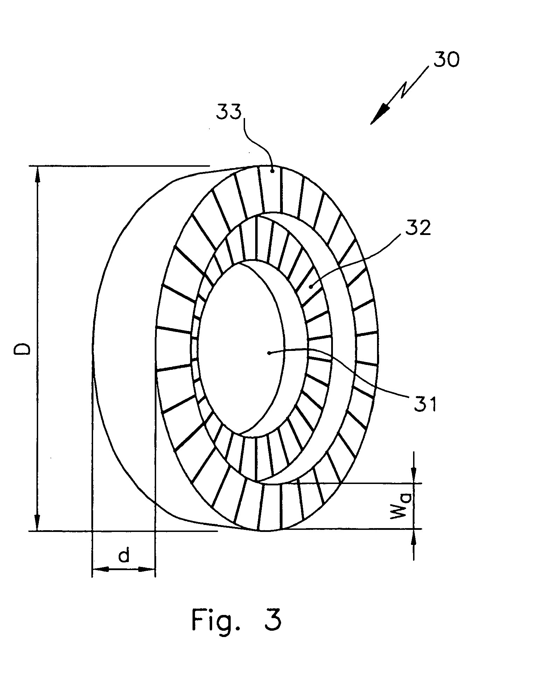

Ohne Beschränkung der Allgemeinheit sind in den Figuren 2a, 2b und 2c für einen Wandler mit einem kreisförmigen zentralen Element und zwei ringförmigen Elementen die Schallfelder für -6 dB, -9 dB und -12 dB für die drei Frequenzen $f\_1$ = 5,2 MHz, f2 =2,1 MHz und $f\_3$ = 1,1 MHz angegeben. Eine Prinzipdarstellung eines solchen Wandlers 30 ist Fig.3 zu entnehmen. Der äußere Durchmesser des Wandlers und damit auch des äußeren Ringes beträgt ebenfalls D = 10 mm. Die Ringbreiten betragen für den äußeren Ring $w\_a$ = 2,5 mm, für den mittleren Ring $w\_m$ = 1,5 mm und der Durchmesser des kreisförmigen Zentralelements beträgt 2 mm. Dem äußeren Ring $R\_a$ wurde die niedrigste Frequenz 1,1 MHz zugeordnet, dem mittleren Ring $R\_m$ die mittlere Frequenz 2,1 MHz und dem Zentralelement die höchste Frequenz 5,2 MHz. Die Dicke der Piezo-Kristalle nimmt für die einzelnen Ring-Elemente frequenzabhängig nach innen mit wachsender Frequenz ab, wie Fig.3 zeigt. Die erzielten schallferder haben für die angegebenen Frequenzen ab einer bestimmten Entfernung zur Wandlerfläche die gleiche Abstrahlcharakteristik im Fernfeld und erfüllen somit in dieser Ausführungsform die Anforderung nach einer empfangsseitigen homogenen Wellenfront.

Die angegebene Frequenzkombination ist aber nicht solitär. Für einen jeweils vorgegebenen Durchmesser der gesamten Annular-Array-Anordnung existieren eine Vielzahl von Frequenzkombinationen mit entsprechenden Ringbreiten und Durchmessern von Zentralelementen, die die Forderung nach einer für die ausgewählten Frequenzen gleich bleibenden Gesamtabstrahlcharakteristik erfüllen. Beispielsweise haben die Schallfelder für o. g. Wandler mit $\varnothing$ = 10 mm von $f\_4$ =4,8 MHz, $f\_5$ = 1,9 MHz und $f\_6$ = 1,0 MHz als Gruppe oder $f\_7$ =5,6 MHz, $f\_8$ =2,3 MHz und $f\_9$ = 1,2 MHz als Gruppe die jeweils gleiche Gesamtabstrahl-Charakteristik. Bei Verwendung einer anderen Ausführungsform des Wandlers mit einem Gesamtdurchmesser $\varnothing$ = 12mm ergeben sich wiederum völlig andere Frequenzkombinationen mit jeweils gleicher Gesamtabstrahl-Charakteristik, z. B. für $f\_10$ = 3,8 MHz, $f\_11$ = 1,95 MHz und $f\_12$ = 1 MHz.

Generell ist die Anzahl der Ringe und damit der verwendbaren Frequenzen nur durch die Herstellungstechnologie beschränkt. Die Ausführungsformen der Wandler mit ringförmigen Elementen sind alle dadurch gekennzeichnet, dass die niedrigen Frequenzen dem oder den äußersten Ring(en) zugeordnet werden, die mittleren Frequenzen dem oder den mittleren Ring(en) und die höchste(n) Frequenz(en) dem kreisförmigen Zentralelement. Die Ringe können unterschiedliche Ringbreiten besitzen, die fest vorgegeben sein können, wenn in dieser Ausführungsform explizit nur eine Frequenz pro Ring verwendet werden soll und der Ring eine dementsprechende Dicke aufweist. Für flexiblere Anwendungen können sich vorgegebene Ringgruppen (mit fester Ringgruppenbreite) aus einer bestimmten Anzahl von Subelementen (Einzelringen mit fester Ringbreite) zusammengesetzt werden, die variabel zu "virtuellen" Ringen mit geeigneter jeweiliger "virtueller" Ringbreite durch Zusammenfassung benachbarter Einzelringe kombiniert werden können. Hierdurch ist es möglich, dass einer Ringgruppe ein ganzes Frequenzband innerhalb bestimmter geeigneter Intervallgrenzen zugeordnet werden kann, beispielsweise die Frequenzbänder $F\_1$ := {1 MHz -1,49 MHz}, $F\_2$ := {1,5 MHz - 1,99 MHz} bis z.B. $F\_8$ := {4,5 MHz - 4,99 MHz} und $F\_9$ := {5 MHz - 6 MHz}.

Da bei dieser Ausführungsform die Dicken der Piezo-Kristalle für die Einzelringe innerhalb einer Ringgruppe gleich bleiben müssen, da ansonsten die "virtuellen Ringe" nicht gebildet werden könnten, nimmt man einen sich im Fernfeld ergebenden Fehler aufgrund entsprechender Verschiebungen der Gesamtabstrahl-Charakteristiken für Frequenzen aus *dieser* Frequenzgruppe in Kauf. Die Abweichungen fallen für die oben genannten, den Ringgruppen zugeordneten schmalen Frequenzbänder noch relativ gering aus. Als Vorteil erhält man die freie Kombinierbarkeit einer Vielzahl von Frequenzen in einem Wandler mit ringförmigen Elementen und die freie Konfigurierbarkeit der zugeordneten Ringe bzw. "virtuellen" Ringe mit geeigneten Ringbreiten, Reihenfolgen und ggf. Wiederholungen von Ringen und deren Frequenzen. Damit ist man in der Lage, bei Verwendung ganzer Frequenzbänder einerseits die Gesamtabstrahl-Charakteristik mit einem tolerierbaren Fehler für alle in der Ultraschall-Sende- und -Empfangseinheit geeignet verwendeten Frequenzkombinationen gleichförmig zu halten und andererseits durch die hiermit erreichte Flexibilität eine Vielzahl von Anwendungsmöglichkeiten zu eröffnen. Diese können im Bereich der zerstörungsfreien Materialprüfung wie auch in der Detektion von Eigenschaften von Medien (Substanzen) liegen, die z. B. in der Medizin, der Umwelt- oder der Energietechnik von hohem Interesse sind, beispielsweise die Detektion gelöster Gase in flüssigen Medien.

Die rechnerische Bestimmung der jeweiligen Schallfelder kann hierbei auf zweierlei Weise erfolgen. Unter Anwendung des Huygens-Prinzips, dass jeder Punkt auf einer sich fortpflanzenden Wellenfront als eine sekundäre Quelle für die Ausbreitung einer Kugelwelle betrachtet werden kann, ergibt sich zwingend, dass die lineare Überlagerung der Anteile aller emittierten Kugelwellen von allen Punktquellen an einem *bestimmten* Punkt im Ausbreitungsraum der emittierten Kugelwellen das Feld an diesem Punkt bestimmt. Daher ist es möglich, durch numerische Integration des Rayleigh-Integrals

$$\Phi\ (\mathbf{r},t) = \iint S\ \text{vn}\ (\mathbf{r0},\ t - r/c)\ /\ (2*\pi * \mathbf{r})\ dS$$

das Feld für jeden Punkt im relevanten Ausbreitungsraum der von den Punktquellen emittierten Kugelwellen zu bestimmen. Hierbei bezeichnet $\Phi$ das Geschwindigkeitspotential für Abstände $\mathbf{r}$ zum Zeitpunkt $\mathbf{t}$, vn bezeichnet die Wellengeschwindigkeit auf der Oberfläche des Transducers normal zur Wandler-Fläche und c die Ausbreitungsgeschwindigkeit im Medium. Mit s (x, y, z, t) hat man ein generelles skalares Feld gegeben, welches im akustischen Fall den akustischen Druck, definiert für einen bestimmten Punkt in Raum und Zeit beschreibt. Für jeden Punkt im Ausbreitungsraum werden die jeweiligen Beiträge aller Punktquellen, die die Wandleroberfläche diskret überdecken, bestimmt und lokal addiert/subtrahiert. Dieses Verfahren ist robust und nur durch die seitens der Anzahl der Punktquellen bedingte Rechenzeit beschränkt.

Die zweite Möglichkeit besteht in der Bestimmung der Impulsantwort h(r,t), die mittels einer Faltung mit der Wellengeschwindigkeit gemäß

$$\Phi\ (\mathbf{r},t) = v(t) \times h\ (\mathbf{r},t)$$

und

$$h(\mathbf{r},t) = \iint (\delta(t - \mathbf{r}/c - t0) \, / \, (2{*}\pi{*}\mathbf{r}) \, dS0$$

das Geschwindigkeitspotential ergibt. Die Impulsantwort für einen bestimmten Punkt im Feld wird phänomenologisch nach der Stepanishen-Methode bestimmt, wobei nur diejenigen Punkte in einer bestimmten Distanz r zum lokalen Feldpunkt zum lokalen Feld beitragen, d. h. die auf einem Kreis um die Projektion des Feldpunkts auf den Wandler liegen. Der Nachteil dieser Methode liegt darin, dass die Form des Wandlers keinen Einfluss auf die Ermittlung der Impulsantwort hat und dass ein homogenes Medium unterstellt ist.

**Patentansprüche**

1. Ultraschall-Sende- und -Empfangseinheit mit mindestens einem Wandler zur Erzeugung von Mehrfrequenz-Ultraschallsignalen und zum Empfang von durch ein Untersuchungsmedium beeinflussten, insbesondere reflektierten, gedämpften und/oder abgelenkten Ultraschallsignalen, wobei der Wandler ein zentrales, kreisförmig ausgebildetes Element und mindestens ein weiteres, um das zentrale Element herum und konzentrisch zu diesem angeordnetes ringförmiges Element aufweist, wobei die Elemente Ultraschallsignale unterschiedlicher Frequenz erzeugen und gleichzeitig emittieren und die vom Untersuchungsmedium beeinflussten Ultraschallsignale aller Frequenzen empfangen und die daraus gebildeten Empfangssignale einer Auswerteeinrichtung zuführen, **dadurch gekennzeichnet, dass** bei vorgegebenem Gesamtdurchmesser des Wandlers der Durchmesser des zentralen kreisförmigen Elements und die Breite der ringförmigen Elemente sowie die Frequenzen der von den einzelnen Elementen emittierten Ultraschallsignale derart gewählt sind, dass das Fernfeld des resultierenden Schallfeldes in homogener Weise und unter Vermeidung von Fokussierungen von allen verwendeten Ultraschallsignalen mit unterschiedlichen Frequenzen überdeckt ist.

2. Ultraschall-Sende- und -Empfangseinheit nach Anspruch 1, **dadurch gekennzeichnet, dass** die Elemente die Ultraschallsignale kontinuierlich emittieren.

3. Ultraschall-Sende- und -Empfangseinheit nach Anspruch 1 oder 2, **dadurch gekennzeichnet, dass** die äußeren Elemente Ultraschallsignale einer niedrigeren Frequenz emittieren als das zentrale Element.

4. Ultraschall-Sende- und -Empfangseinheit nach einem der vorhergehenden Ansprüche, **dadurch gekennzeichnet, dass** mehrere ringförmige Elemente vorgesehen sind, deren Breite und/oder deren gegenseitiger Abstand variiert.

5. Ultraschall-Sende- und -Empfangseinheit nach einem der Ansprüche 1 bis 4, **dadurch gekennzeichnet, dass** die ringförmigen Elemente mindestens teilweise in dicht benachbarte Einzelringe aufgeteilt sind, die jeweils Ultraschallsignale geringer Frequenzdifferenz emittieren.

6. Ultraschall-Sende- und -Empfangseinheit nach einem der Ansprüche 1 bis 5, **dadurch gekennzeichnet, dass** die Dicke der ringförmigen Elemente größer ist als die Dicke des zentralen Elements.

7. Ultraschall-Sende- und -Empfangseinheit nach einem der vorhergehenden Ansprüche, **dadurch gekennzeichnet, dass** die Elemente Piezoelemente sind.

8. Ultraschall-Sende- und -Empfangseinheit nach einem der vorhergehenden Ansprüche, **dadurch gekennzeichnet, dass** eine optische Empfangseinrichtung vorgesehen ist.

**Claims**

1. Ultrasound transmitting and receiving unit with at least one converter for producing multi-frequency ultrasound signals and for receiving ultrasound signals that have been influenced, particularly reflected, attenuated and/or deflected by a test medium, in which the converter has a central, circular element and at least a further ring shaped element that is arranged around the central element and concentrically to it, in which the elements produce ultrasound signals of varying frequency and at the same time transmit and receive ultrasound signals of all frequencies that have been influenced by the test medium and take the signals received that have been formed from them to an evaluation device, **characterised in that** with the preset overall diameter of the converter the diameter of the central circular element and the width of the ring shaped element as well as the frequencies of the ultrasound signals transmitted by the individual elements are selected in such a way that the far field of the resulting sound field is covered by all ultrasound signals with different frequencies that are used in a homogenous way with the prevention of focussing.

2. Ultrasound transmitting and receiving unit according to claim 1, **characterised in that** the elements transmit the ultrasound signals continuously.

3. Ultrasound transmitting and receiving unit according to claim 1 or 2, **characterised in that** the external elements transmit ultrasound signals of a lower frequency than the central element.

4. Ultrasound transmitting and receiving unit according to one of the preceding claims, **characterised in that** several ring shaped elements are provided, the width and/or distance from each other of which varies.

5. Ultrasound transmitting and receiving unit according to one of claims 1 to 4, **characterised in that** the ring shaped elements are at least partially split up into individual rings that are close to each other, which transmit ultrasound signals with a small frequency difference.

6. Ultrasound transmitting and receiving unit according to one of claims 1 to 5, **characterised in that** the thickness of the ring shaped elements is larger than the thickness of the central element.

7. Ultrasound transmitting and receiving unit according to one of the preceding claims, **characterised in that** the elements are piezo elements.

8. Ultrasound transmitting and receiving unit according to one of the preceding claims, **characterised in that** an optical receiving device is provided.

**Revendications**

1. Unité d'émission et de réception d'ultrasons avec au moins un convertisseur destinée à la génération de signaux à ultrasons multifréquences et à la réception de signaux à ultrasons influencés, en particulier réfléchis, amortis et/ou déviés par un support d'analyse, dans laquelle le convertisseur présente un élément central, conçu en forme de cercle, et au moins un autre élément en forme d'anneau, disposé autour de l'élément central et de manière concentrique par rapport à celui-ci, dans laquelle les éléments génèrent et transmettent simultanément des signaux à ultrasons de différentes fréquences et reçoivent les signaux à ultrasons de toutes les fréquences, influencés par le support d'analyse, et alimentent une unité d'évaluation avec les signaux de réception qui en résultent, **caractérisée en ce que**, pour un diamètre total prédéterminé du convertisseur, le diamètre de l'élément central, conçu en forme de cercle, et la largeur des éléments en forme d'anneau, ainsi que les fréquences des signaux à ultrasons émis par chacun des éléments sont sélectionnés de telle sorte que le champ lointain du champ acoustique en résultant est masqué de manière homogène par différentes fréquences et en évitant les concentrations de tous les signaux à ultrasons employés.

2. Unité d'émission et de réception d'ultrasons selon la revendication 1, **caractérisée en ce que** les éléments transmettent les signaux à ultrasons en continu.

3. Unité d'émission et de réception d'ultrasons selon la revendication 1 ou 2, **caractérisée en ce que** les éléments extérieurs transmettent des signaux à ultrasons à une fréquence plus basse que l'élément central.

4. Unité d'émission et de réception d'ultrasons selon l'une des revendications précédentes, **caractérisée en ce que** plusieurs éléments en forme d'anneau sont prévus, dont la largeur et/ou la distance mutuelle de les uns par rapport aux autres varient.

5. Unité d'émission et de réception d'ultrasons selon l'une des revendications 1 à 4, **caractérisée en ce que** les éléments en forme d'anneau sont répartis, au moins en partie, en anneaux individuels étroitement contigus, qui transmettent respectivement les signaux à ultrasons à une amplitude de fréquence plus basse.

**6.** Unité d'émission et de réception d'ultrasons selon l'une des revendications 1 à 5, **caractérisée en ce que** l'épaisseur des éléments en forme d'anneau est supérieure à l'épaisseur de l'élément central.

**7.** Unité d'émission et de réception d'ultrasons selon l'une des revendications précédentes, **caractérisée en ce que** les éléments sont des éléments piézoélectriques.

**8.** Unité d'émission et de réception d'ultrasons selon l'une des revendications précédentes, **caractérisée en ce qu'**un dispositif de réception optique est prévu.

Fig. 1

EP 2 271 439 B1

Fig. 2a

FF = Inf [mm], f = 2.1 [MHz], osc = Inf atten=0.01978 [dB/cm/MHz]

Fig. 2b

FF = Inf [mm], f = 5.2 [MHz], osc = Inf atten=0.01257 [dB/cm/MHz]

BEAMWIDTH [mm], Aperture (AZ) 2 mm

Range in [mm], Azimuth focus=20 [mm]    Envelope

Fig. 2c

Fig. 3

Fig. 4a

Fig. 4b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 29708338 U1
- US 4569231 A
- US 4437348 A
- DE 19732968 A1